# EUROPEAN PATENT APPLICATION

(11) **EP 4 311 563 A1**
(43) Date of publication of application: **31.01.2024**
(21) Application number: 23187204.5
(22) Date of filing: 24.07.2023
(51) Int. Cl.: A61M 5/00, A61J 1/16, B01L 9/06, B65B 43/54, B65D 25/10

(54) **SUPPORT DEVICE FOR SUPPORTING A PLURALITY OF CONTAINERS FOR PHARMACEUTICAL USE TO BE HANDLED IN A PROCESSING LINE**

(30) Priority: 25.07.2022 IT 202200015591
(71) Applicant: NUOVA OMPI S.r.l. Unipersonale, 35017 Piombino Dese (PD) (IT)
(72) Inventor: CANESTRARO, Marco, 35010 Carmignano di Brenta (PD) (IT)
(74) Representative: Porta & Consulenti Associati S.p.A.

(57) **Abstract**

The invention relates to a support device (10) for supporting a plurality of containers for pharmaceutical use to be handled in a processing line. The support device (10) comprises a plate (20) having a plurality of housing seats (30) configured to house said containers, each housing seat (30) comprising a support base (32a) configured to support said containers and a side wall (34) extended from said base (32a) along a longitudinal direction (Y). The base (32) comprises at least three rest elements (32a) projecting inwardly in said housing seat (30) and equally spaced from each other. The side wall (34) comprises a plurality of centring ribs (38) extended along said longitudinal direction (Y) and projecting inwardly in the housing seat (30). A pair of centring ribs (38) is arranged above a respective rest element (32a).

## Description

The present invention relates to a support device for supporting a plurality of containers for pharmaceutical use to be handled in a processing line.

The support device of the present invention meets the definition of "nest" of ISO 21881:2019 standard.

Although explicit reference will be made in this description and the following claims to containers for pharmaceutical use, the support device of the invention is also suitable for supporting containers for medical, cosmetic or veterinary use.

For simplicity of discussion, in the present description all the containers that be supported by the support device of the invention are also indicated with the term "primary containers".

Non-limiting examples of primary containers are vials, ampoules, cartridges and syringes.

In the present description and in the appended figures specific reference will be made, as a non-limiting example of primary containers, to the cartridges.

As known, the primary containers are often handled in the processing lines by aggregating them into a secondary container called "nest".

In accordance with ISO 21881:2019 standard, the nest comprises a plate made of plastic material on which a plurality of housing seats configured to house primary containers are made. These housing seats are distributed in the plate according to a predetermined layout and are shaped so as to stably support the primary containers, keeping them separated from each other and thus avoiding possible mutual collisions.

It is also known to aggregate the primary containers into a so-called "nest and tub" configuration, in which the primary containers are placed inside the housing seats of a nest and the nest is in turn placed inside a tray so-called "tub". This configuration is particularly suitable for supporting and handling the primary containers during the various processing steps to which they are subjected along the processing line, such as washing, packaging, sterilization, filling, capping, crimping, freeze-drying, storage, manual or automated inspection operations, etc.

US 10207832 B2 describes a nest in which each housing seat comprises a base defined by four rest elements configured to support a respective cartridge and a side wall from which eight centring ribs for centring the cartridges extend into the housing seats. The rest elements are equally spaced from each other. Analogously, the centring ribs are equally spaced from each other. A centring rib is arranged at a central zone of a respective rest element and another centring rib is arranged between each pair of adjacent rest elements.

The Applicant has noted that a sufficient number of centring ribs should be provided within each housing seat in order to ensure a stable positioning of the primary containers in the respective housing seats of the nest. According to the Applicant, such centring ribs must be at least six.

The Applicant therefore deems that a solution such as the one described in US 10207832 B2, by providing in each housing seat eight centring ribs equally spaced from each other, actually allows to achieve a stable positioning of the cartridges within the housing seats.

However, the Applicant has noted that such stable positioning would inevitably be compromised if one of the abovementioned centring ribs is damaged or not correctly sized. In this case, in fact, the two centring ribs that are adjacent to the damaged or incorrectly sized centring rib would be sufficiently distant from each other and would therefore not be able to prevent displacements of the cartridge towards the side wall portion in which the damaged or incorrectly sized centring rib is provided.

The technical problem underlying the present invention is to provide a support device suitable for ensuring a stable positioning of the primary containers within the housing seats even in the case that one of the centring ribs typically provided in the housing seats is damaged or not correctly sized.

The present invention therefore relates to a support device for supporting a plurality of containers for pharmaceutical use to be handled in a processing line, comprising a plate having a plurality of housing seats configured to house said containers, wherein each housing seat comprises a base configured to support said containers and a side wall extended from said base along a longitudinal direction, said base comprising at least three rest elements projecting inwardly in said housing seat and equally spaced from each other, said side wall comprising a plurality of centring ribs extended along said longitudinal direction and projecting inwardly in said housing seat, characterised in that said plurality of centring ribs comprises a pair of centring ribs arranged above a respective rest element.

In the present description and in the appended claims, the terms "upper" and "lower", and similar terms such as "above" and/or "below" and/or "over" and/or "under", if present, are used with reference to the position taken by the support device during handling in the processing line of the containers for pharmaceutical use arranged therein.

Thanks to the provision of two centring ribs at each rest element, each centring rib is in close proximity to another centring rib. In this way, if one of the abovementioned centring ribs is damaged or not correctly sized, the centring action is still effectively carried out by the other of the abovementioned centring ribs, thus ensuring in this way the desired stable positioning of the container inside the housing seat.

The Applicant also believes that the provision of two centring ribs above each rest element provides the support device with a greater structural stiffness than in the case where only one centring rib is provided on each rest element.

The present invention can have at least one of the preferred features recited in the dependent claims and described hereinbelow, wherein each of these features can be provided individually or in combination with the other preferred features.

Preferably, said centring ribs are arranged only above said at least three rest elements.

The Applicant has observed that this arrangement is particularly functional during the operations of insertion/extraction of the containers into/from the housing seats, both in the case of manual insertion/extraction and in the case of mechanized insertion/extraction. The Applicant has indeed found that during the abovementioned operations it is necessary to be able to access the spaces defined between pairs of adjacent rest elements and, since there are no centring ribs in the support device of the invention at the abovementioned spaces, access to said spaces is clear of obstacles or obstructions.

Preferably, each rest element comprises a first side connected to the side wall.

Preferably, each rest element comprises a second side arranged inside said housing seat in a distal position with respect to said side wall.

Preferably, the second side is shorter than the first side.

Preferably, each rest element comprises two opposite third sides connecting said first side to said second side.

Preferably, each centring rib of each pair of centring ribs is arranged at a respective third side of the respective rest element.

Therefore, the two centring ribs provided at each rest element are at a distance from each other substantially equal to the length of the abovementioned first side.

Preferably, each centring rib is connected to the respective rest element. In this way each pair of centring ribs strengthens the respective rest element.

Preferably, each centring rib comprises, at one of the free ends thereof that is distal from the respective rest element, a tapered portion.

This tapered portion facilitates the insertion of the container into the housing seat.

Preferably, said centring ribs extend on opposite sides with respect to said plate.

Preferably, said rest elements are four or six.

Preferably, said pairs of centring ribs are four or six.

Preferably, said plate comprises a flat surface which is perforated at said housing seats.

Preferably, said longitudinal direction is substantially orthogonal to said plane surface.

Further characteristics and advantages of the present invention will become clearer from the following detailed description of preferred embodiments thereof, which is made with reference to the accompanying drawings and given for indicative and non-limiting purposes. In such drawings:
- Figure 1 is a schematic perspective top view of a first embodiment of a support device in accordance with the present invention;
- Figure 2 is a schematic perspective bottom view of the support device of Figure 1;
- Figure 3 is a schematic perspective view, in section and on an enlarged scale of a portion of the support device of Figure 1;
- Figure 4 is a schematic perspective view on an enlarged scale of a further portion of the support device of Figure 1;
- Figure 5 is a schematic perspective view on an enlarged scale of a portion of a second embodiment of a support device in accordance with the present invention;
- Figure 6 is a schematic perspective view on an enlarged scale of a portion of a third embodiment of a support device in accordance with the present invention;
- Figure 7 is a schematic perspective view on an enlarged scale of a portion of a further embodiment of a support device in accordance with the present invention;
- Figure 8 is a plan bottom view of the support device of Figure 7;
- Figure 9 is a schematic perspective bottom view and on an enlarged scale of a portion of the support device of Figure 6;
- Figure 10 is a schematic perspective bottom view of a further embodiment of a support device in accordance with the present invention.

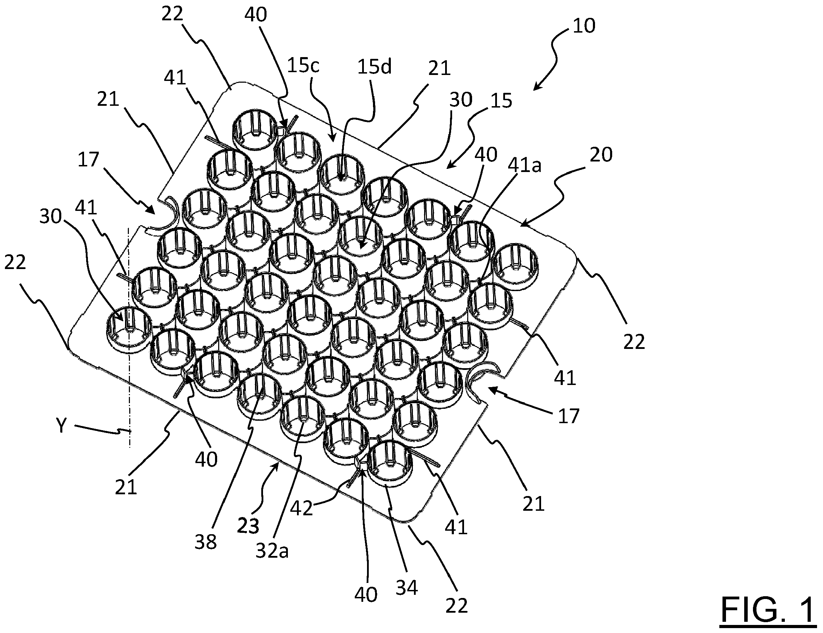

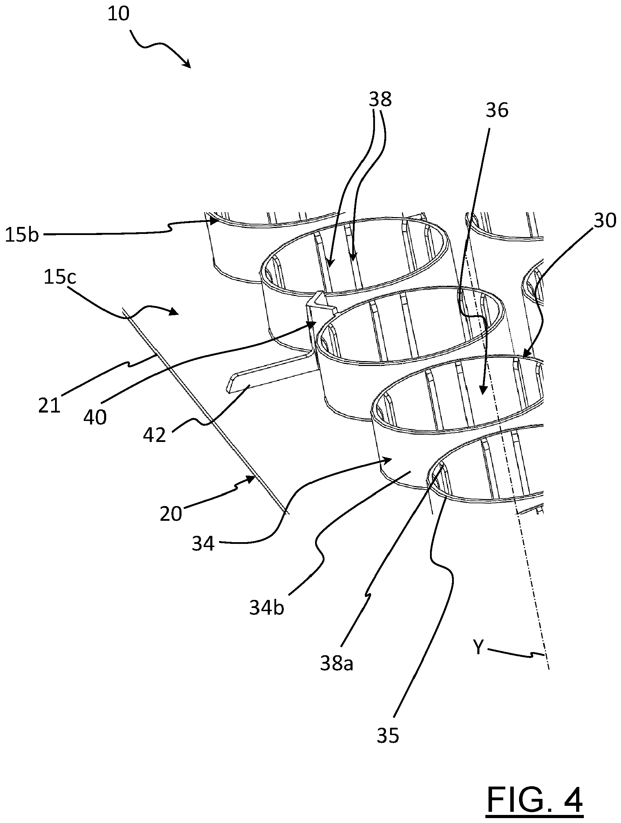

In Figures 1-4, a first embodiment of a support device for supporting a plurality of containers for pharmaceutical use (not shown) in accordance with the present invention is indicated overall with 10.

These containers are intended to be handled in a processing line (not shown).

Preferably, the abovementioned containers are cartridges.

The support device 10 comprises a body 15 having a substantially quadrangular shape. In the non-limiting example described herein, the body 15 is defined by a plate 20 having a substantially rectangular shape. Said plate 20 has four sides 21 and four vertices 22, which are preferably rounded.

The plate 20 of the support device 10 can be made by moulding plastic material, for example polypropylene.

The plate 20 comprises a flat surface which is perforated at a plurality of housing seats 30 configured to house respective cartridges.

The housing seats 30 are distributed in the plate 20 according to a predetermined layout and are shaped so as to stably support the cartridges, keeping them separated from each other and thus avoiding possible mutual collisions.

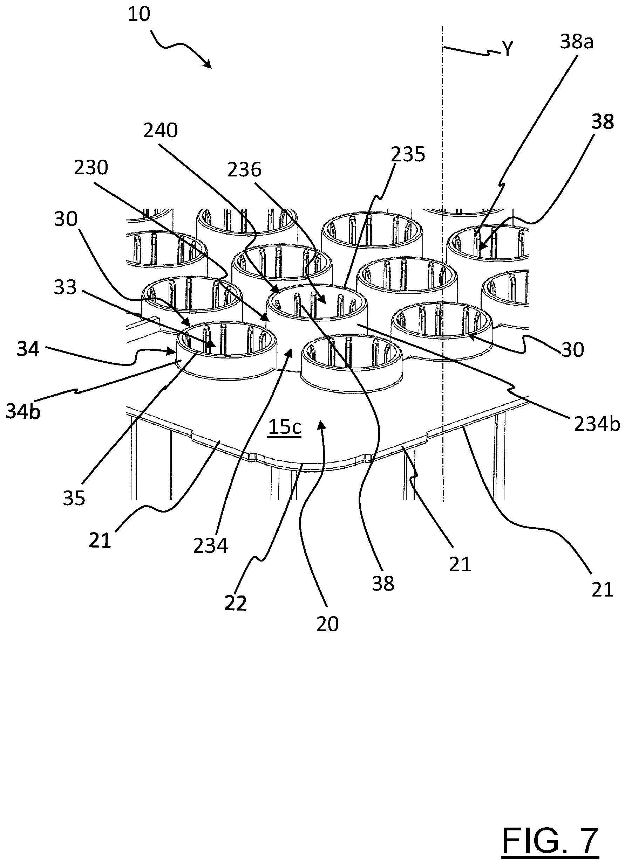

In the non-limiting example of Figures 1-4 the housing seats 30 are arranged in the plate 20 along a plurality of parallel rows (or columns) (Figure 1). The housing seats 30 may however be arranged according to a staggered honeycomb or checkerboard configuration, like for example in Figures 7 to 10.

A plurality of housing seats 30 are adjacent to the sides 21 of the plate 20 and are hereinafter referred to as "perimeter housing seats 30", while another plurality of housing seats 30 are arranged in the plate 20 in a central position with respect to the perimeter housing seats 30 and are hereinafter referred to as "central housing seats 30".

The body 15, and hence the plate 20, has a perimeter portion 15c delimited externally by the four sides 21 and by the four vertices 22 of the plate 20, and a central portion 15d delimited by the perimeter portion 15c and comprising the perimeter housing seats 30 and the central housing seats 30. The four sides 21 and the four vertices 22 form a perimeter edge 23 of the plate 20.

Each cartridge has a substantially cylindrical shape and comprises a substantially circular open or closed base surface and, on the opposite side to the base surface, a top surface provided with a through opening configured to allow the filling of the cartridge with a predetermined amount of a pharmaceutical product, in the exemplary and non-limiting case of cartridges for pharmaceutical use.

The cartridges are typically made of glass or of a thermoplastic material.

After having placed the cartridges of a production batch in the housing seats 30 of the support device 10 with their top surface turned downwards, an organized assembly of cartridges is obtained, that allows the simultaneous handling and processing of all the cartridges of the production batch.

The cartridges, in fact, upon being placed in the housing seats 30 of the support device 10, are moved along the processing line by handling the support device 10. At least part of this handling is carried out after having inserted the support device 10 into a tub (not shown).

To this end, the perimeter portion 15c of the plate 20 comprises a pair of opposite through slots 17 configured to facilitate the insertion and the extraction of the support device 10 into/from the tub.

The tub has a substantially parallelepipedal or truncated-pyramidal shape and comprises a substantially flat bottom surface and four substantially flat side walls which are connected two by two by respective connecting walls.

An inner perimeter edge extends from the inner faces of the side walls and of the connecting walls inwardly in the tub. This inner perimeter edge defines an abutment surface on which the perimeter portion 15c of the plate 20 is intended to rest when the support device 10 is placed inside the tray. The inner perimeter edge may be replaced by a plurality of ribs extending from the inner faces of the side walls and/or connecting walls, each rib comprising a rest surface defining a respective abutment surface on which the perimeter portion 15c of the plate 20 is intended to be rested.

Once the cartridges of a production batch are inserted into the housing seats 30 of the support device 10 and the latter is inserted into the tub, an aggregation configuration of the cartridges known as "nest and tub" is made, in which the support device 10 is the "nest" and the tub is the "tub".

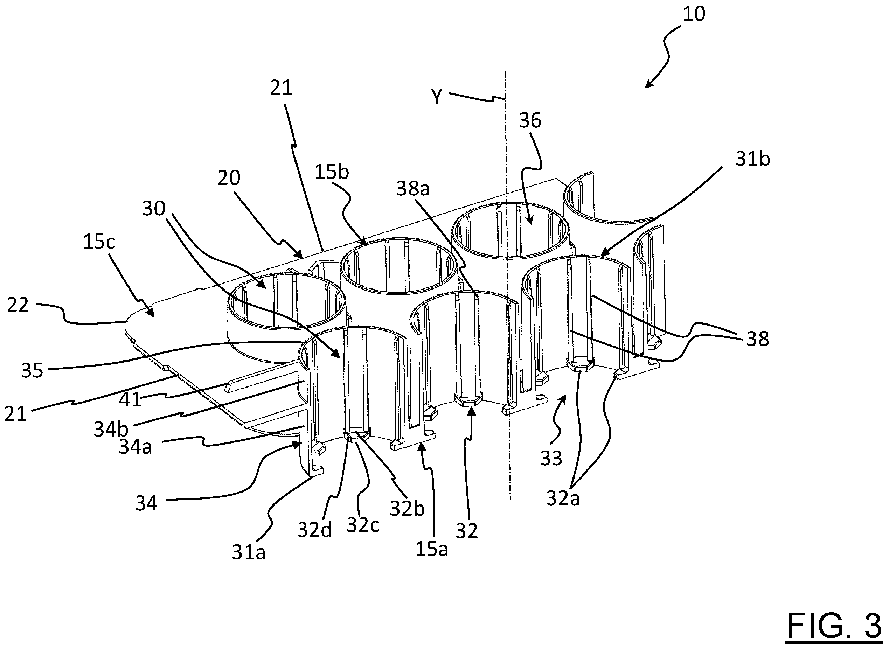

As shown for example in Figure 3, the housing seats 30 extend on opposite sides of the plate 20 between a lower surface 15a arranged below the plate 20 and an upper surface 15b that, in the specific example shown in the accompanying drawings, is arranged above the plate 20 but that in other embodiments can be defined by the upper face of the plate 20.

The lower surface 15a is substantially flat and is defined at lower ends 31a of the housing seats 30.

The upper surface 15b is also substantially flat and is defined at the upper ends 31b of the housing seats 30.

Each of the housing seats 30 comprises, at the lower end thereof 31a, a base 32 having a through opening 33 and, at the upper end thereof 31b, an end edge 35 defining a through opening 36 configured to allow the insertion and the extraction of a respective cartridge. The end edges 35 of the housing seats 30 project above the plate 20, all at the same height.

The base 32 has a predetermined thickness measured between a lower face of the base 32 that is configured to rest on a substantially horizontal operating surface of the processing line and that lies on the abovementioned lower surface 15a and an upper face of the base 32 that is configured to support the cartridge.

Each housing seat 30 further comprises a side wall 34 which, in the non-limiting example of Figures 1-4, is substantially cylindrical and delimits a substantially cylindrical space configured to receive the cartridge.

The side wall 34 extends starting from the base 32 up to the upper surface 15b of the plate 20 along a longitudinal axis Y that is substantially orthogonal to the abovementioned flat surface of the plate 20.

As shown for example in Figure 3, the side walls 34 extend on opposite sides with respect to the plate 20. In this way, a lower portion 34a of the side wall 34 is placed below the plate 20 (i.e. on the side of the base 32) and an upper portion 34b of the side wall 34 is placed above the plate 20 (i.e. on the side of the upper end 31b).

In the non-limiting example of Figures 1-4, the base 32 is defined by six rest elements 32a projecting radially from the side wall 34 inwardly in the housing seat 30 and circumferentially equally spaced from each other, in particular arranged angularly offset by 60° from each other around the longitudinal axis Y. In other embodiments not shown, the rest elements are four and are angularly offset from each other by 90° or three and are angularly offset from each other by 120°.

In the present description and in the appended claims, the terms "axial" and the like are used to refer to a longitudinal direction of the housing seats 30 that is parallel to the longitudinal axis Y, the terms "radially" and the like are used to refer to any direction perpendicular to the abovementioned longitudinal axis Y, while the terms "circumferentially" and the like are used to refer to any circumferential direction about the abovementioned longitudinal axis Y.

Each rest element 32a has a quadrangular shape, substantially trapezoidal, and comprises a first side 32b connected to the side wall 34, a second side 32c arranged inside the housing seat 30 in a distal position with respect to the side wall 34 and preferably shorter than the first side 32b and two opposite third sides 32d connecting the first side 32b to the second side 32c.

To allow centring the cartridges within the respective housing seats 30, each housing seat 30 comprises a plurality of centring ribs 38 projecting radially from the tubular wall 34 inwardly in the housing seat 30 and extending along a longitudinal direction parallel to the longitudinal axis Y.

In the non-limiting example of Figures 1-4, the centring ribs 38 are twelve and have the same shape and dimensions as each other.

In general, the number of the centring ribs 38 is preferably even, so as to have a certain number of pairs of centring ribs 38, which in the non-limiting example of Figures 1-4 is six pairs.

Each pair of centring ribs 38 is arranged at and above a respective rest element 32a of the base 32.

The pairs of centring ribs 38 are circumferentially equally spaced from each other.

In the non-limiting example of Figures 1-4, the centring ribs 38 are arranged only above the rest elements 32a, so that in the circumferential spaces defined between pairs of adjacent rest elements 32a no centring ribs 38 are provided.

In particular, the centring ribs 38 of each pair of centring ribs 38 are arranged at the two third sides 32d of the respective rest element 32a.

In the non-limiting example of Figures 1-4, the centring ribs 38 extend on opposite sides with respect to the plate 20 between the rest elements 32a and the upper ends 31b of the housing seats 30.

Each centring rib 38 comprises, at a free end thereof that is distal from the respective rest element 32a, a tapered portion 38a configured to facilitate the insertion of the cartridge into the housing seat 30.

The support device 10 comprises a plurality of stacking elements 40 configured to allow stacking on the support device 10 another support device 10 of the same type.

In the non-limiting example of Figures 1-4, the stacking elements 40 are four and are arranged near two opposite sides 21 of the plate 20. The four stacking elements 40 are arranged substantially symmetrically with respect to a transverse centreline plane of the plate 20. In other embodiments that are not shown, the stacking elements 40 could be more or less than four, for example two or six.

Each stacking element 40 projects above the end edges 35 of the housing seats 30, and thus above the upper surface 15b.

In the non-limiting example of Figures 1-4, the stacking elements 40 are made in a single piece with the plate 20 and extend between the side walls 34 of two adjacent perimeter housing seats 30, in particular between the upper portions 34b of said two housing seats 30.

Each stacking element 40 is shaped like a "V", with the vertex of the "V" facing toward the perimeter edge 23.

Each stacking element 40 is connected, at the vertex of the "V", to a reinforcing rib 42 also arranged above the plate 20 and preferably made in a single piece with the stacking element 40 and the plate 20.

Further reinforcing ribs are arranged above the plate 20. In the non-limiting example of Figures 1-4, four further ribs 41 are arranged at the perimeter portion 15c, two of which are connected to the upper portions 34b of two perimeter housing seats 30. Further reinforcing ribs 41a are arranged at the central portion 15d between two respective adjacent housing seats 30.

In the preferred embodiment illustrated herein, the plate 20 comprises, on the lower surface 15a, one or more recesses 44 formed between two respective adjacent housing seats 30 configured to house respective stacking elements of another support device 10 of the same type.

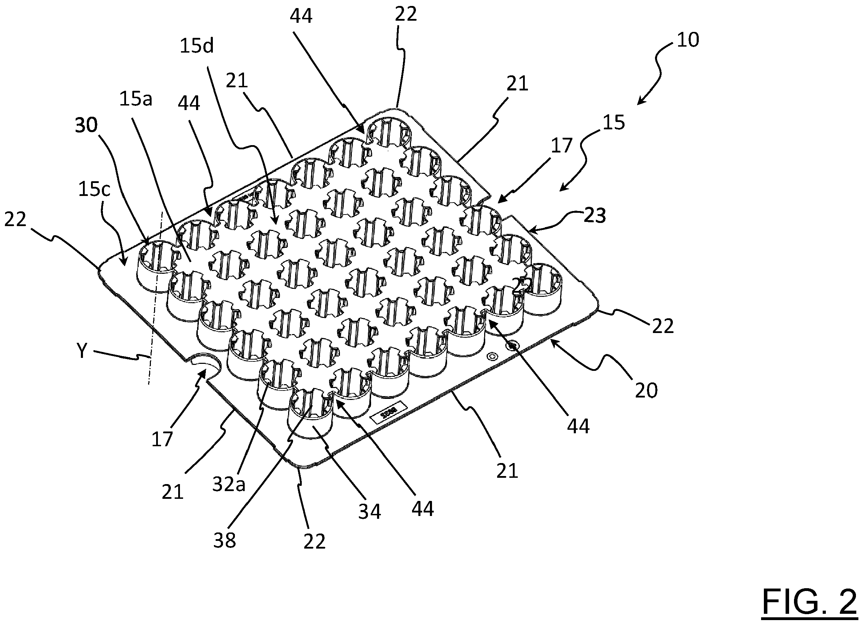

In particular, four recesses 44 are provided, each configured to house one of the abovementioned four stacking elements 40 (Figure 2). Each recess 44 is defined between two lower portions 34a of the side walls 34 of two adjacent perimeter housing seats 30. The abovementioned recesses 44 are open towards the perimeter edge 23 of the plate 20.

The support device 10 described above can therefore be arranged both under another support device of identical type and over another support device of identical type, defining in both cases an assembly (or stack) of support devices which, thanks to the provision of stacking elements housed in respective recesses, is particularly stable.

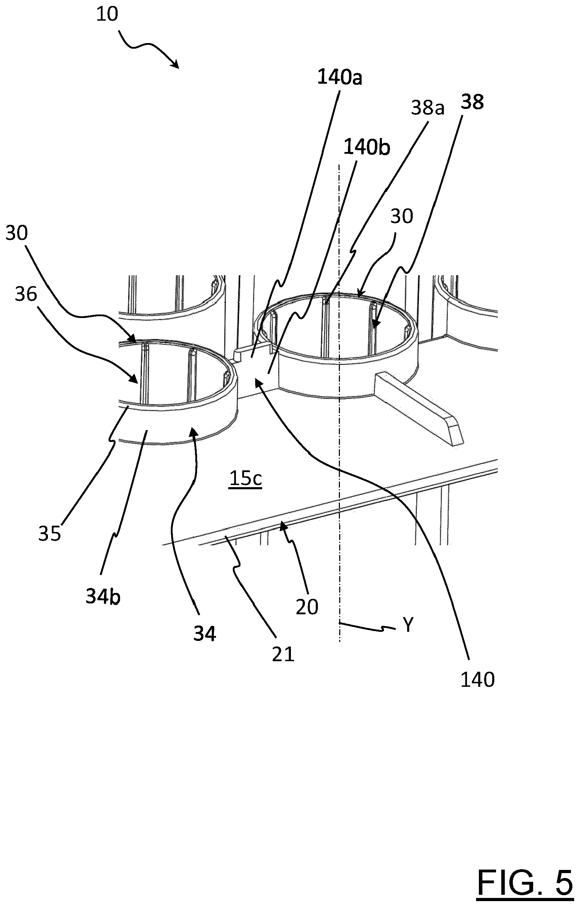

A second embodiment of a support device 10 in accordance with the present invention is shown in Figure 5. Parts and components that are identical or analogous to those of the support device 10 of Figures 1-4 are indicated by the same reference numeral and for their description reference is made to the above description.

The support device 10 of Figure 5 differs from the support device 10 of Figures 1-4 in the stacking elements, indicated with 140, which in this case have a cross-section shaped like an "I" and which do not have respective reinforcing ribs analogous to the reinforcing ribs 42 described above with reference to Figures 1-4.

Each stacking element 140 comprises an upper portion 140a projecting above the end edges 35 of the housing seats 30, and therefore above the upper surface 15b, and that has a transverse length, measured along a direction orthogonal to the longitudinal axis Y, which is lower than the distance between the two housing seats 30 connected by the stacking element 140. Said upper portion 140a extends from a lower portion 140b of the stacking element 140 that extends between the plate 20 and the end edges 35 and that connects the abovementioned two housing seats 30. The transverse length of the lower portion 140b is therefore greater than that of the upper portion 140a.

In the non-limiting example of Figure 5, four pairs of centring ribs 38, arranged above four rest elements (not shown) are provided.

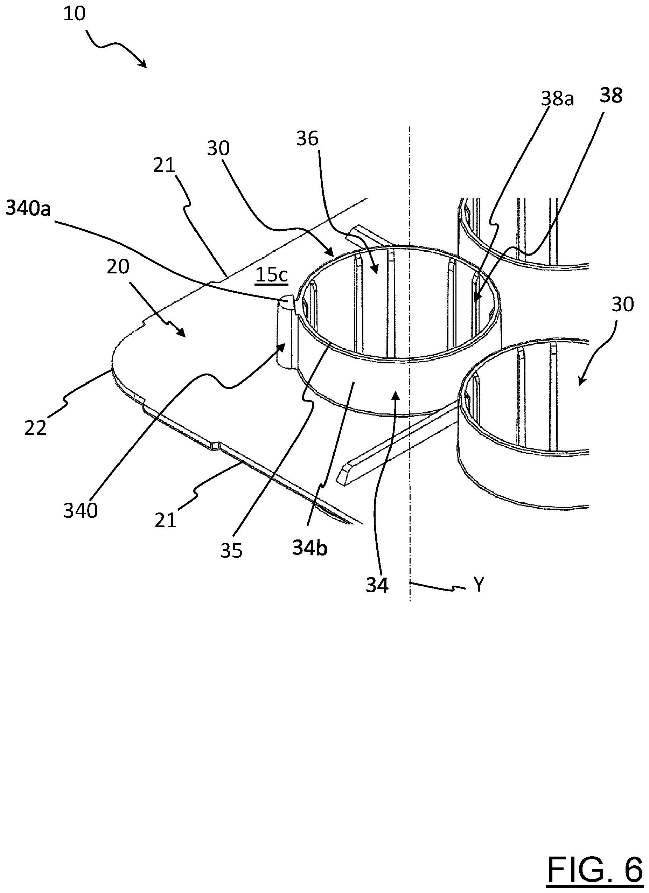

A third embodiment of a support device 10 in accordance with the present invention is shown in Figure 6. Parts and components that are identical or analogous to those of the support device 10 of Figures 1-4 are indicated by the same reference numeral and for their description reference is made to the above description.

Looking at the plate 20 of the support device 10 of Figure 6 from above, it can be noted that it differs from the support device 10 of Figures 1-4 in the stacking elements, that in this case are defined by stacking elements 340 extending outwardly from the upper portion 34b of the side wall 34.

Each of the stacking elements 340 comprises a respective upper portion 340a projecting above the end edges 35 of the housing seats 30.

In particular, in the embodiment of Figure 6, each of the four perimeter housing seats 30 arranged at the four vertices 22 of the plate 20 comprises a respective stacking element 340 facing toward the perimeter portion 15c of the plate 20 and having a substantially semicylindrical shape.

A further embodiment of a support device 10 in accordance with the present invention is shown in Figures 7-9. Parts and components that are identical or analogous to those of the support device 10 of Figures 1-4 are indicated by the same reference numeral and for their description reference is made to the above description.

Looking at the plate 20 of the support device 10 of Figures 7-9 from above, it can be noted that it differs from the support device 10 of Figures 1-4 both in the arrangement of the housing seats 30, which in this case is staggered like a honeycomb or checkerboard, and in the stacking elements 240, which in this case are defined by end edges 235 of housing seats 230 projecting above the end edges 35 of the other housing seats 30 of the plate 20.

The housing seats 230 differ from the other adjacent housing seats 30 only in that their upper portion 234b of their side wall 234 has an extension greater than that of the upper portion 34b of the side wall 34 of the other housing seats 30.

The centring ribs 38 of the housing seats 230 are identical to the centring ribs 38 of the housing seats 30, whereby the centring ribs 38 of the housing seats 230 do not reach the end edges 235 of the housing seats 230.

The housing seats 230 are four, arranged near two opposite sides 21 of the plate 20 in a substantially symmetrical manner with respect to a transverse centreline plane of the plate 20.

Looking at the plate 20 of the support device 10 of Figures 7-9 from below, it can be noted that it differs from the support device 10 of Figures 1-4 also in that a plurality of annular-shaped recesses 244 are made on the lower surface 15a of the plate 20.

The recesses 244 are four and are arranged at the four housing seats 230.

Each recess 244 extends circumferentially around the base 32 of the respective housing seat 230. Each base 32 of the housing seats 230 is identical to the base 32 of the housing seats 30 and comprises six rest elements 32a configured to support a respective cartridge. The lower face of the rest elements 32a of the housing seats 230 is defined on the lower surface 15a of the plate 20. The depth of the recesses 244 is lower than the thickness of the rest elements 32a.

The four recesses 244 are configured to house the end edges 235 of another support device 10 identical to that of Figures 7-9 so as to stack those two support devices 10.

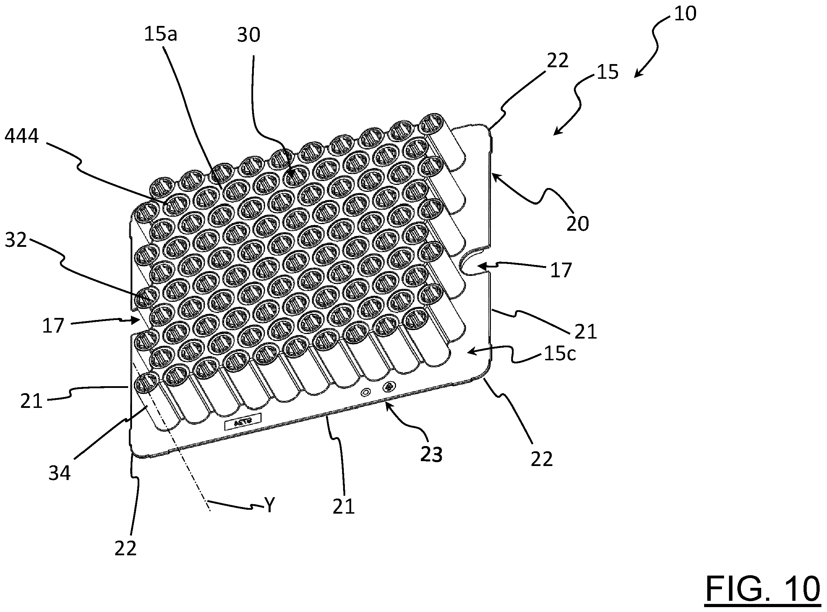

A further embodiment of a support device 10 in accordance with the present invention is shown in Figure 10. Parts and components that are identical or analogous to those of the support device 10 of Figures 1-4 are indicated by the same reference numeral and for their description reference is made to the above description.

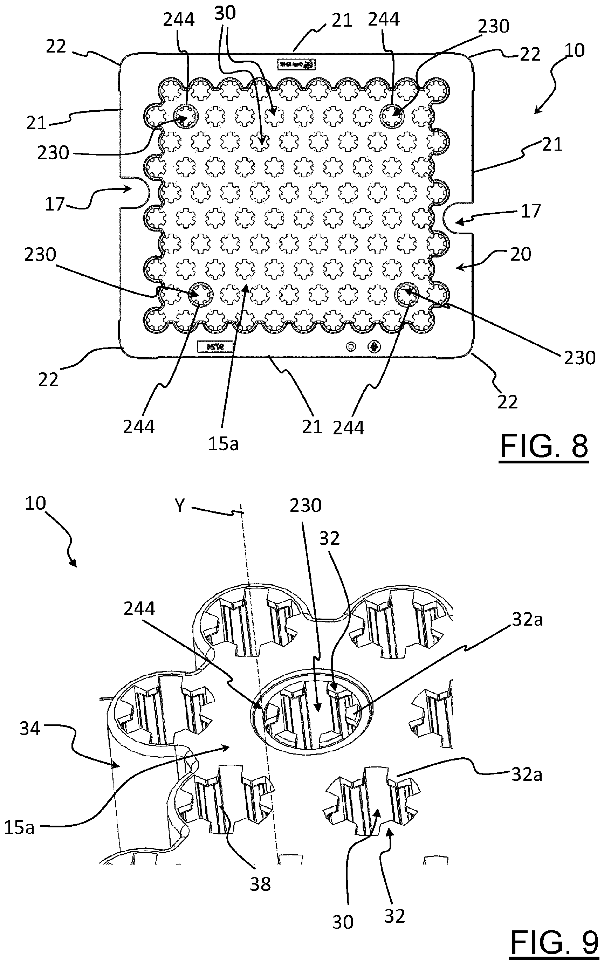

The support device 10 of Figure 10 differs from the support device 10 of Figures 1-4 in that the stacking elements 40 are not provided and in that annular-shaped recesses 444 totally analogous to the recesses 244 of Figures 8 and 9 are made at all the housing seats 30.

Each base 32 of the housing seats 30 of Figure 10 is identical to the base 32 of the housing seats 30 of Figures 1-4. Thus, the lower face of the base 32 of the housing seats 30 of Figure 10 is defined on the lower surface 15a of the plate 20.

The recesses 444 are configured to house the end edges 35 of a support device 10 identical to that of Figure 10 so as to stack said two support devices 10. The support device 10 provided with the abovementioned recesses 444 can therefore be arranged above or below another support device 10 of the same type.

Obviously, in order to meet specific and contingent needs, a person skilled in the art will be able to make numerous modifications and variants to the invention described above, all of which falling within the scope of protection defined by the following claims.

## Claims

1. Support device (10) for supporting a plurality of containers (50) for pharmaceutical use to be handled in a processing line, comprising a plate (20) having a plurality of housing seats (30, 230) configured to house said containers (50), wherein each housing seat (30, 230) comprises a base (32) configured to support said containers (50) and a side wall (34, 234) extended from said base (30, 230) along a longitudinal direction (Y), said base (30, 230) comprising at least three rest elements (32a) projecting inwardly in said housing seat (30, 230) and equally spaced from each other, said side wall (34, 234) comprising a plurality of centring ribs (38) extended along said longitudinal direction (Y) and projecting inwardly in said housing seat (30, 230), **characterised in that** said plurality of centring ribs (38) comprises a pair of centring ribs (38) arranged above a respective rest element (32a).

2. Support device (10) according to claim 1, wherein said centring ribs (38) are arranged only above said at least three rest elements (32a).

3. Support device (10) according to claim 1 or 2, wherein each rest element (32a) comprises a first side (32b) connected to the side wall (34, 234), a second side (32c) arranged inside said housing seat (30, 230) in a distal position with respect to said side wall (34, 234) and two opposite third sides (32d) connecting said first side (32b) to said second side (32c), wherein each centring rib (38) of each pair of centring ribs (38) is arranged at a respective third side (32d) of the respective rest element (32a).

4. Support device (10) according to any one of the previous claims, wherein each centring rib (38) is connected to the respective rest element (32a).

5. Support device (10) according to any one of the previous claims, wherein each centring rib (38) comprises, at one of the free ends thereof that is distal from the respective rest element (32a), a tapered portion (38a).

6. Support device (10) according to any one of the previous claims, wherein said centring ribs (38) extend on opposite sides with respect to said plate (20).

7. Support device (10) according to any one of the previous claims, wherein said rest elements (32a) and said pairs of centring ribs (38) are four or six.

8. Support device (10) according to any one of the previous claims, wherein said plate (20) comprises a flat surface which is perforated at said housing seats (30, 230) and said longitudinal direction (Y) is substantially orthogonal to said plane surface.
